# EUROPÄISCHE PATENTSCHRIFT

(11) **EP 2 925 393 B1**
(45) Veröffentlichungstag und Bekanntmachung des Hinweises auf die Patenterteilung: **11.01.2017**
(21) Anmeldenummer: 13820716.2
(22) Anmeldetag: 29.11.2013
(51) Int. Cl.: A61B 5/15, A61B 5/154, A61M 5/32

(54) **SICHERHEITSBAUGRUPPE FÜR DIE MEDIZINTECHNIK**
SAFETY ASSEMBLY FOR MEDICAL TECHNOLOGY
GROUPE DE SÉCURITÉ POUR LA TECHNIQUE MÉDICALE

(30) Priorität: 29.11.2012 AT 505492012
(43) Veröffentlichungstag der Anmeldung: 07.10.2015
(73) Patentinhaber: Greiner Bio-One GmbH, 4550 Kremsmünster (AT)
(72) Erfinder: EBETSBERGER, Franz, A-4550 Kremsmünster (AT); KOFLER, Georg, A-4565 Inzersdorf (AT)
(74) Vertreter: Burger, Hannes
(86) Internationale Anmeldenummer: PCT/AT2013/050230
(87) Internationale Veröffentlichungsnummer: WO 2014/082111

(56) Entgegenhaltungen:
- EP-A1- 1 587 419
- EP-A2- 0 623 359
- DE-A1-102005 054 989

## Beschreibung

Die Erfindung betrifft eine Sicherheitsbaugruppe in der Medizintechnik für die Blutabnahme wie diese im Anspruch 1 beschrieben wird.

Die DE 10 2005 054 989 A1 bescheribt eine Blutentnahmevorrichtung mit einem an seinem hinteren Ende gegenüber einem Entnahmeröhrchen festlegbaren Nadelhalter, gegenüber dessen vorderen Ende eine Kanüle und ein die Kanüle nach deren Gebrauch abdeckender Nadelschutz befestigt sind. Der Nadelschutz ist dabei aus einem aus einer Grundposition in eine Position über der Kanüle verschwenkbaren Abdeckabschnitt gebildet. Der schwenkbare Abdeckabschnitt ist mit einem Befestigungsabschnitt gelenkig verbunden, wobei der Befestigungsabschnitt an einem über eine Stirnwand vorragenden zapfenartigen Ende drehbar gelagert und an einer an diesem angeordneten Verriegelungsschulter in Axialrichtung verriegelt gehalten ist. An einander zugewandten Flächen des Befestigungsabschnittes des Nadelschutzes und dem zapfenartigen Ende des Nadelhalters sind zwischen diesen in Radialrichtung wirkende Rastelemente vorgesehen. Dabei weist der Befestigungsabschnitt des Nadelschutzes eine zentrale Öffnung mit einer innenseitig daran angebrachten Riffelung auf. Diese Riffelung wirkt mit mindestens einem gegenüberliegend angeordneten Steg des Nadelhalters korrespondierend zusammen. Damit kann eine relative Positionierung des Nadelschutzes bezüglich des Nadelhalters festgelegt werden. Nachteilig dabei ist, dass es bei jeder relativen Verlagerung zwischen dem Befestigungsabschnitt und dem zapfenartigen Ende des Nadelhalters zu einer Aufweitung des Lagersitzes kommt und dies in manchen Betriebsfällen zu Störungen, wie einem Verklemmen oder einem ungewollten Ablösen, führen kann.

Die EP 0 623 359 A2 bzw. die dazu parallel laufende GB 2 277 685 A beschreiben eine Sicherheitsbaugruppe für die Blutabnahme, mit einer Handhabungsvorrichtung mit einem hohl ausgebildeten Hauptkörperteil, welcher ein distales Ende mit einem Halteansatz und ein offen ausgebildetes proximales Ende aufweist. Eine Schutzvorrichtung umfasst einen ringförmigen Basisteil sowie ein mit dem Basisteil schwenkbar verbundenes Schutzelement, welches von einer Freigabestellung in eine Schutzstellung verschwenkbar ist. Damit kann in der Schutzstellung eine an der Handhabungsvorrichtung angebrachte Nadelanordnung zumindest teilweise abgedeckt werden. Der Basisteil ist am Halteansatz der Handhabungsvorrichtung um die Längsachse drehbar gehalten. Weiters ist eine Rastvorrichtung mit zumindest einem ersten Rastelement an der Handhabungsvorrichtung sowie zumindest einem zweiten Rastelement an der Schutzvorrichtung vorgesehen, wobei bei in Eingriff stehenden ersten und zweiten Rastelementen eine relative Position der Schutzvorrichtung bezüglich der Handhabungsvorrichtung festgelegt ist. Das zumindest erste an der Handhabungsvorrichtung vorgesehene Rastelement ist bezüglich des Halteansatzes in radialer Richtung bezüglich der Längsachse distanziert vom Halteansatz sowie in radialer Richtung bezüglich er Längsachse auf der von der Längsachse abgewendeten Seite des Basisteils angeordnet ist. Weiters sind am Hauptkörperteil an seiner Außenfläche über die Außenfläche vorragende weitere Rastelemente angeordnet, in welche in der rohrförmigen Aufsteckteilwand ausgebildete Rastausnehmungen in einer Raststellung eingreifen. Bei verrasteter Stellung des Aufsteckteils am Hauptkörperteil kann diese Raststellung nur durch eine gegenläufige Verdrehbewegung um die Längsachse gelöst werden, um in einer anderen Rastposition neuerlich verrastet werden zu können.

Aus der WO 2004/066840 A1 ist eine Handhabungsvorrichtung, ein daran kuppelbares Einstichelement und eine schwenkbare Schutzvorrichtung zur Abdeckung nach dem Gebrauch bekannt geworden. Zwischen Halteelementen der Schutzvorrichtung und der Handhabungsvorrichtung ist eine Positioniervorrichtung angeordnet, wobei diese bei in Eingriff stehenden Halteelementen eine Schwenkebene eines Schutzelementes der Schutzvorrichtung in Bezug zur Handhabungsvorrichtung in ihrer Lage festlegt. Kupplungselemente einer Kupplungsvorrichtung zwischen der Handhabungsvorrichtung und dem Einstichelement sind zueinander derart ausgebildet, dass bei vollständig gekuppelter Stellung der beiden Kupplungselemente eine kürzere Öffnungsachse einer Öffnung am proximalen Ende des Einstichelementes etwa parallel zur Schwenkebene oder in der Schwenkebene verlaufend ausgerichtet ist. Nachteilig dabei ist die starre Positionierung der schwenkbaren Schutzvorrichtung bezüglich der Handhabungsvorrichtung.

Aus der US 5,139,489 A ist ein Gehäusehalter zur Aufnahme eines Aufriahmeröhrchens bekannt geworden, welcher an einer vom Innenraum abgewendeten Seite einen Fortsatz mit einem daran angeordneten Außen- sowie Innengewinde aufweist. Mit dem Außengewinde des Fortsatzes ist ein Basisteil der schwenkbar ausgebildeten Schutzvorrichtung mittels eines darin angeordneten Innengewindes aufschraubbar. Dieser Basisteil weist weiters in dessen Zentrum einen Durchbruch auf, der in seiner Lage mit dem im Fortsatz angeordneten Innengewinde lagemäßig korrespondiert. In das Innengewinde des Fortsatzes des Gehäusehalters ist die Nadelanordnung einschraubbar. Zu diesem Zweck ist das Schutzgehäuse um eine Schwenkachse ausgehend von der durch den Gehäusehalter verlaufenden Längsachse wegzuschwenken, um diesen Einschraubvorgang durchführen zu können. Eine exakt vorbestimmbare Ausrichtung des Nadelendes in Bezug auf die Schwenkebene des Schutzgehäuses konnte dabei nicht immer sichergestellt werden.

Aus der EP 0 812 597 A2 sind andere Ausbildungsmöglichkeiten einer Schutzvorrichtung für eine Nadelanordnung bekannt geworden, bei welcher entweder der Basisteil der Schutzvorrichtung mittels der Nadelanordnung schwenkbar um die Längsachse des Halters an diesem gehalten oder der Basisteil des Schutzelements auf die Nadelanordnung aufgesetzt bzw. auch mit dieser verbunden ist. Die so geschaffene Einheit ist über einen Normanschluss mit einem Spritzengehäuse oder einem Gehäusehalter für Blutentnahmeröhrchen verbindbar.

Aus der US 5,277,311 A sowie der US 5,312,369 A sind jeweils Schutzvorrichtungen für Nadelanordnungen bekannt geworden, bei welchen der Basiskörper des schwenkbar ausgebildeten Schutzelements der Schutzvorrichtung dreh- bzw. schwenkbar am Fortsatz des Gehäusehalters bzw. der Handhabungsvorrichtung angeordnet ist. Zusätzlich können dabei zwischen dem Fortsatz und dem Basisteil reibungserhöhende Bauteile vorgesehen sein, um die aufzubringende Kraft festzulegen, welche notwendig ist, um den Basisteil der Schutzvorrichtung relativ zur Längsachse um den Fortsatz verdrehen zu können. Das Schutzelement ist über verformbar ausgebildete Teile bzw. Gelenke von einer Freigabestellung der Nadelanordnung hin in eine Schutz- bzw. Abdeckstellung verschwenkbar.

Aus der US 3,658,061 A ist eine medizinische Baugruppe, beispielsweise für die Blutabnahme, bekannt geworden, die ein Handhabungsvorrichtung, ein daran gehaltertes bzw. eingesetztes Einstichelement sowie eine schwenkbare Schutzvorrichtung für zumindest einen Teilbereich des Einstichelements umfasst. Die Schutzvorrichtung ist bedarfsweise lösbar, jedoch feststehend an der Handhabungsvorrichtung gehaltert, wobei ein flexibler Teil zwischen dem Schutzelement und dem Halteelement der Schutzvorrichtung eine Gelenkanordnung ausbildet. Die Schutzvorrichtung ist von einer ersten, das Einstichelement freigebenden, in eine zweite, das Einstichelement abdeckende Stellung verschwenkbar. Das Schutzelement weist einen Kanal zur Aufnahme eines Teilbereiches des Einstichelements auf, wobei dieser Kanal derart ausgebildet ist, dass mittels Reibschluss bzw. Klemmung das Schutzelement in der Schutzstellung am Einstichelement gehalten ist.

Ein weiteres Schutzelement ist aus der US 4,664,259 A bekannt geworden, bei welchem im Bereich des Schutzelements in der eingeklappten Stellung, also der Schutzstellung, dem Schutzelement ein Rastelement zugeordnet mit welchem das Schutzelement in der Schutzstellung am Einstichelement, insbesondere der Hohlnadel, verrastet gehalten werden kann, um so ein unbeabsichtigtes, neuerliches Freigeben des proximalen Endes des Einstichelementes zu verhindern bzw. zu vermeiden.

Aus der EP 0 626 924 B1 ist eine andere Schutzvorrichtung für eine medizinische Baugruppe bekannt geworden, bei der das Schutzelement bedarfsweise lösbar an der Handhabungsvorrichtung anbringbar ist. Dabei ist das Halteelement der Schutzvorrichtung in Richtung der Längsachse, also in axialer Richtung der Handhabungsvorrichtung feststehend an dieser gehaltert, wobei jedoch eine Drehung des Halteelements mitsamt dem Schutzelement um die Längsachse möglich ist.

Weitere schwenkbare Nadelschutzvorrichtungen sind aus der EP 0 702 973 B1 bzw. der EP 0 885 621 B1 bekannt geworden, bei welchen jeweils das Halteelement für das Schutzelement der Schutzvorrichtung an einem von der Handhabungsvorrichtung abgewendeten Bereich des Einstichelementes an diesem gehalten ist. Dadurch wird eine Baueinheit geschaffen, bei welcher das Einstichelement zumeist gemeinsam mit dem Schutzelement in die Handhabungsvorrichtung einzusetzen ist. Ähnlich ausgestaltete Schutzvorrichtungen sind aber auch noch aus der US 6,436,086 B1, US 6,440,104 B1, US 2002/0151852 A1, US 2002/0151853 A1, US 2002/0156425 A1, US 2002/0156427 A1, US 2002/0161336 A1 sowie der US 2002/0193744 A1 bekannt geworden.

Der vorliegenden Erfindung liegt die Aufgabe zugrunde, eine Sicherheitsbaugruppe für die Medizintechnik zu schaffen, die eine einwandfrei, frei verdrehbare Lagerung der Schutzvorrichtung an der Handhabungsvorrichtung aufweist und trotzdem eine verrastbare Lagepositionierung der Schutzvorrichtung relativ bezüglich der Handhabungsvorrichtung möglich ist.

Diese Aufgabe der Erfindung wird durch die Merkmale des Anspruches 1 gelöst. Der sich durch die Merkmale des Anspruches 1 ergebende Vorteil liegt darin, dass so eine räumlich getrennte Anordnung der Lagerung bzw. Halterung des Basisteils der Schutzvorrichtung am Halteansatz der Handhabungsvorrichtung von der Rastvorrichtung zwischen diesen Bauteilen erfolgt. Durch diese räumliche Trennung der Lagerstelle von der Rastvorrichtung kann so jede Einheit für sich exakt aufeinander abgestimmt werden, wodurch eine noch sicherere Funktion für die relativen Verlagerungsvorgänge zueinander erzielbar ist. So kann je nach verwendetem Werkstoff für die Schutzvorrichtung sowie die Handhabungsvorrichtung der Lagersitz entsprechend den Einsatzbedingungen ausgebildet werden, um damit eine einwandfreie Verstell- bzw. Verschwenkbewegung um die Längsachse am Halteansatz durchführen zu können. Unabhängig davon kann dann die Rastkraft der Rastvorrichtung zwischen den Rastelementen festgelegt werden, ohne dass dabei der Lagersitz beeinträchtigt ist. Damit kann aber auch ein gegenseitiges Verklemmen zwischen den weiterhin relativ zueinander verdrehbaren Bauteilen verhindert werden. Darüber hinaus können einerseits die Haltekraft in der Raststellung sowie andererseits die für die Verdrehung notwendige Verstellkraft zur Überwindung der zwischen den Rastelementen aufgebauten Rastkraft festgelegt werden. Die freie Drehbarkeit am Halteansatz bleibt davon unberührt. Weiters kann damit aber auch eine gewisse Anpresskraft des Basisteils an den Halteansatz erzielt werden. Damit kann die Schutzvorrichtung mit ihrem Basisteil je nach Anzahl sowie Anordnung der Rastelemente zwischen den einzelnen vorgesehenen Raststellungen um den den Basisteil lagernden Halteansatz verdreht werden.

Vorteilhaft ist auch eine weitere Ausführungsform nach Anspruch 2, da dadurch über den Umfang mehrere Raststellungen zwischen der Schutzvorrichtung und der Handhabungsvorrichtung festgelegt werden können und so eine noch individuellere Anpassung an die persönlichen Bedürfnisse des medizinischen Personals in Bezug auf den ordnungsgemäßen Gebrauch erzielbar ist.

Vorteilhaft ist weiters eine Ausbildung nach Anspruch 3, da so ein stabiler Kranz am äußeren Umfangsbereich der Stirnwand geschaffen werden kann, welcher zu einer zusätzlichen Stabilisierung der Handhabungsvorrichtung beiträgt. Darüber hinaus kann so auch eine störungsfreie Verstellung stattfinden, da ein gegenseitiges Verklemmen durch andere hineinragende Teile eher vermieden werden kann.

Durch die Ausbildung nach Anspruch 4 ist es möglich, einen noch besseren Schutz der Rastvorrichtung sowie des am Halteansatz gehaltenen Basisteils der Schutzvorrichtung zu erzielen. Damit können auch Manipulationen erschwert werden, wodurch die gesamte Sicherheit der Sicherheitsbaugruppe erhöht werden kann. Darüber hinaus ist es dadurch auch möglich, je nach gewählter Wandstärke des Kragens die Haltekraft sowie die für die Verdrehung notwendige Verstellkraft exakt vorbestimmen zu können.

Nach einer anderen Ansführungsvariante gemäß Anspruch 5 wird so im Bereich des Halteansatzes eine definierte Abstützebene für den Basisteil geschaffen, wodurch die exakte Führung und damit verbundene Lagerung der Schutzvorrichtung für deren Verstellbewegung um die Längsachse geschaffen wird. Weiters wird damit aber auch eine kompakte Bauweise der Handhabungsvorrichtung erzielt, um so mit einem minimalen Werkstoffaufwand das Auslangen zur Bildung der Sicherheitsbaugruppe zu finden.

Bei der Ausgestaltung nach Anspruch 6 ist von Vorteil, dass so eine symmetrische Verrastung zwischen dem Basisteil und der Handhabungsvorrichtung erzielt werden kann. Darüber hinaus wird so aber auch eine gewisse Zentrierung zusätzlich zur Lagerung am Halteansatz geschaffen. Des Weiteren wird aber auch durch die diametral gegenüberliegenden, schlitzförmig ausgebildeten Ausnehmungen eine gewisse zusätzliche Nachgiebigkeit bzw. Elastizität des Basisteils geschaffen, um einerseits die Aufsatzbewegung auf den Halteansatz und andererseits eine gewisse Verformung des Basisteils für die Verstellbewegung um die Längsachse zu ermöglichen.

Die Aufgabe der Erfindung kann aber eigenständig auch durch die Merkmale des Anspruches 7 gelöst werden. Die sich aus der Merkmalskombination dieses Anspruches ergebenden Vorteile liegen darin, dass so auch hier eine voneinander räumlich getrennte Anordnung der Lagerung bzw. Halterung des Basisteils der Schutzvorrichtung am Halteansatz der Handhabungsvorrichtung von der Rastvorrichtung zwischen diesen Bauteilen erfolgt. Durch diese räumliche Trennung der Lagerstelle von der Rastvorrichtung kann so jede Einheit für sich exakt aufeinander abgestimmt werden, wodurch eine noch exaktere Funktion für die relativen Verlagerungsvorgänge erzielbar ist. So kann je nach verwendetem Werkstoff für die Schutzvorrichtung sowie die Handhabungsvorrichtung der Lagersitz entsprechend den Einsatzbedingungen ausgebildet werden, um so eine einwandfreie Verstell- bzw. Verschwenkbewegung um die Längsachse am Halteansatz durchführen zu können. Unabhängig davon kann dann die Rastkraft der Rastvorrichtung zwischen den Rastelementen durch die axiale Höhe der Rastelemente festgelegt werden, ohne dass dabei die exakte Lagerung betroffen ist. Weiters kann so zwischen der Stirnwand der Handhabungsvorrichtung und dem Basisteil eine auf Scherung wirkende Rastkraft zwischen dem Basisteil und der Stirnwand der Handhabungsvorrichtung geschaffen werden. Darüber hinaus kann so aber ein einfach herzustellender Bauteil geschaffen werden, welcher einfach entformbar und darüber hinaus eine sichere Verrastung zwischen den zusammenwirkenden Bauteilen ermöglicht. Damit kann aber auch ein gegenseitiges Verklemmen zwischen den relativ zueinander verdrehbaren Bauteilen verhindert werden. Weiters kann damit einerseits die Haltekraft in der Raststellung sowie andererseits die für die Verdrehung notwendige Verstellkraft zur Überwindung der zwischen den Rastelementen aufgebauten Rastkraft festgelegt werden.

Vorteilhaft ist auch eine Ausbildung nach Anspruch 8, da so je nach gewählter Raumform des Rastelements einerseits die Rastwirkung bzw. Rastkraft sowie andererseits die für die Verstellung notwendige Verstellkraft einfach an die unterschiedlichen Einsatzbedingungen angepasst werden kann.

Vorteilhaft ist auch eine weitere Ausführungsform nach Anspruch 9, da dadurch über den Umfang mehrere Raststellungen zwischen der Schutzvorrichtung und der Handhabungsvorrichtung festgelegt werden können und so eine noch individuellere Anpassung an die persönlichen Bedürfnisse des medizinischen Personals in Bezug auf den ordnungsgemäßen Gebrauch erzielbar ist.

Dabei erweist sich eine Ausgestaltung nach Anspruch 10 vorteilhaft, da dadurch in der verrasteten und gekuppelten Stellung eine Manipulation durch die gegenseitige, abdeckende Stellung zwischen den Rastelementen verhindert wird. Darüber hinaus können aber auch von au-ßen einwirkende Störeinflüsse auf die Rastvorrichtung weitestgehend ausgeschaltet werden.

Nach einer vorteilhaften Weiterbildung gemäß Anspruch 11 kann so eine symmetrische Verrastung zwischen dem Basisteil und der Handhabungsvorrichtung erzielt werden. Darüber hinaus wird so aber auch eine gewisse Zentrierung zusätzlich zur Lagerung am Halteansatz geschaffen. Des Weiteren wird aber auch durch die diametral gegenüberliegenden, schlitzförmig ausgebildeten Ausnehmungen eine gewisse zusätzliche Nachgiebigkeit bzw. Elastizität des Basisteils geschaffen, um einerseits die Aufsatzbewegung auf den Halteansatz und andererseits eine gewisse Verformung des Basisteils für die Verstellbewegung um die Längsachse zu ermöglichen.

Von Vorteil ist aber auch eine Ausbildung nach Anspruch 12, da dadurch bei eingesetzter Nadelanordnung eine noch exaktere Lagerstelle sowie Halterung für den Basisteil am Halteansatz geschaffen werden kann. Darüber hinaus wird aber auch eine Abnahme der Schutzvorrichtung in allen Betriebsfällen verhindert, um so eine Wiederverwendung auch dann noch zu unterbinden, wenn bereits eine Benutzung der Sicherheitsbaugruppe erfolgt ist.

Schließlich ist aber auch eine Ausbildung, wie im Anspruch 13 beschrieben, möglich, da so ein gewisser, axialer Verstellweg des Basisteils am Halteansatz ermöglicht wird, wobei jedoch in der Raststellung stets eine vordefinierte Halte- bzw. Rastkraft durch das Federelement aufgebaut wird. Weiters kann damit aber auch die aufzubringende Verstellkraft des Basisteils um die Längsachse verringert werden, da das Federelement eine gewisse axiale Längsverstellung des Basisteils am Halteansatz zulässt, wobei nach Erreichen der weiteren, vorgewählten Rastposition wiederum eine ausreichende Haltekraft zur Positionierung der Schutzvorrichtung relativ bezüglich der Handhabungsvorrichtung aufgebaut wird.

Zum besseren Verständnis der Erfindung wird diese anhand der nachfolgenden Figuren näher erläutert.

Es zeigen jeweils in stark schematisch vereinfachter Darstellung:
- Fig. 1: eine Sicherheitsbaugruppe im komplett zusammen gebauten Zustand, in schaubildlicher Darstellung;
- Fig. 2: die Sicherheitsbaugruppe nach Fig. 1 in schaubildlich Darstellung sowie in voneinander distanzierter Position der einzelnen Bauteile;
- Fig. 3: die Handhabungsvorrichtung der Sicherheitsbaugruppe nach den Fig. 1 und 2 mit daran angeordneten ersten Rastelementen, in schaubildlicher Darstellung;

### Fortsetzung auf Seite 9 des Urtextes !

- Fig. 4: die Schutzvorrichtung der Sicherheitsbaugruppe nach den Fig. 1 und 2 mit den zweiten Rastelementen, in schaubildlicher Darstellung;
- Fig. 5: einen Axialschnitt der Sicherheitsbaugruppe nach den Fig. 1 bis 4 im Bereich der Rastvorrichtung;
- Fig. 6: eine weitere mögliche Ausbildung der ersten Rastelemente an einer Handhabungsvorrichtung, in schaubildlicher Darstellung;
- Fig. 7: die Schutzvorrichtung der Sicherheitsbaugruppe für die Handhabungsvorrichtung nach Fig. 6 mit den zweiten Rastelementen, in schaubildlicher Darstellung;
- Fig. 8: einen Axialschnitt der Sicherheitsbaugruppe nach den Fig. 6 und 7 im Bereich der Rastvorrichtung;
- Fig. 9: eine andere Ausbildung der ersten Rastelemente an einer Handhabungsvorrichtung, in schaubildlicher Darstellung.

Einführen sei festgehalten, dass in den unterschiedlich beschriebenen Ausführungsformen gleiche Teile mit gleichen Bezugszeichen bzw. gleichen Bauteilbezeichnungen versehen werden, wobei die in der gesamten Beschreibung enthaltenen Offenbarungen sinngemäß auf gleiche Teile mit gleichen Bezugszeichen bzw. gleichen Bauteilbezeichnungen übertragen werden können. Auch sind die in der Beschreibung gewählten Lageangaben, wie z.B. oben, unten, seitlich usw. auf die unmittelbar beschriebene sowie dargestellte Figur bezogen und sind bei einer Lageänderung sinngemäß auf die neue Lage zu übertragen.

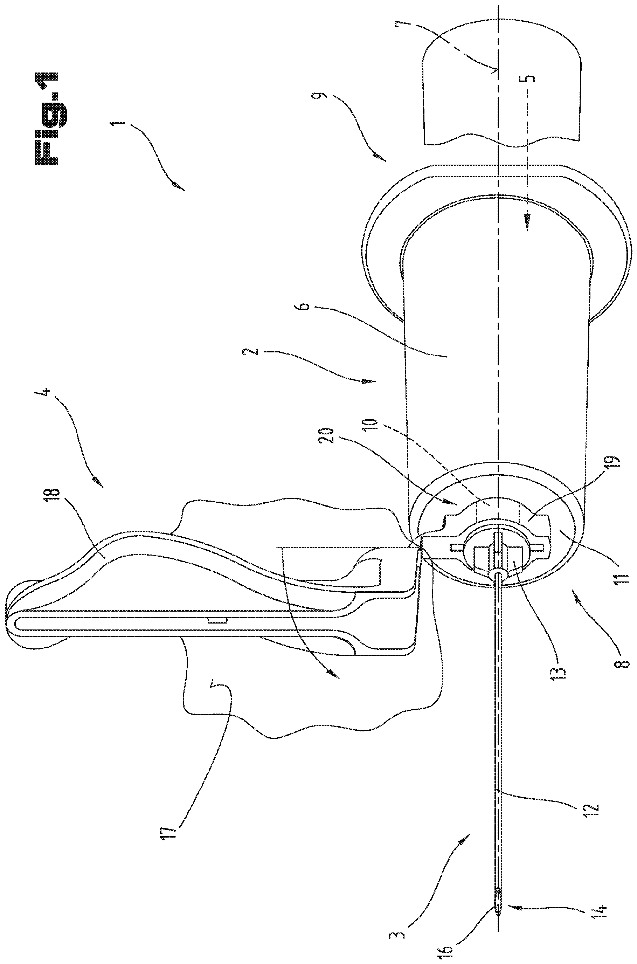

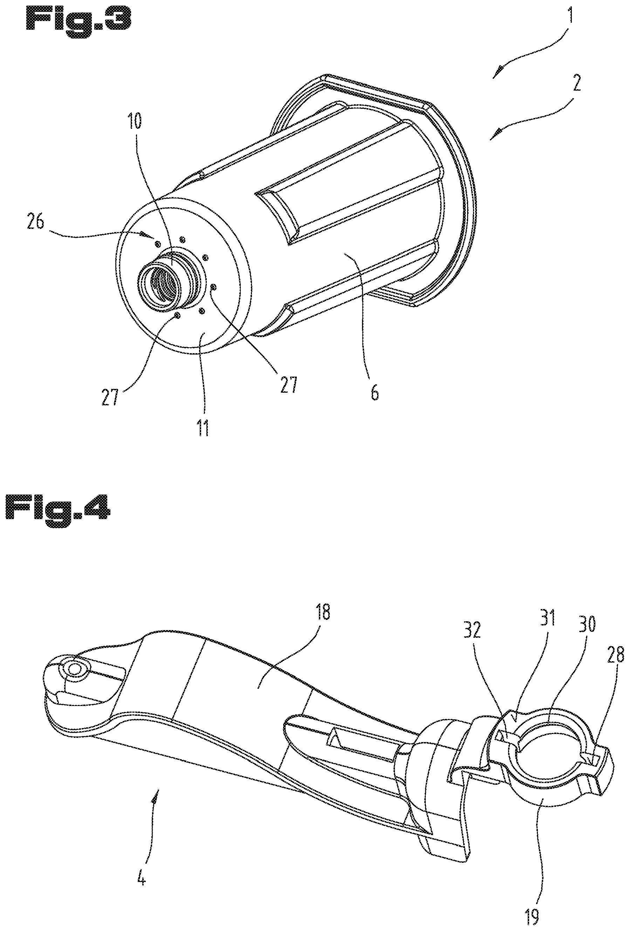

In den Fig. 1 bis 5 ist eine Sicherheitsbaugruppe 1, insbesondere für die Blutabnahme in der Medizintechnik, gezeigt, welche zumindest eine Handhabungsvorrichtung 2, eine daran bedarfsweise halterhares Nadelanordnung 3 sowie eine schwenkbare Schutzvorrichtung 4 für zumindest einen Teilbereich der Nadelanordnung 3 umfasst.

Die Handhabungsvorrichtung 2 kann beispielsweise durch einen eine Aufnahmekammer 5 bildenden bzw. umgrenzenden hohl ausgebildeten Hauptkörperteil 6 gebildet sein, der in Richtung einer Längsachse 7 voneinander distanziert ein distales Ende 8 sowie ein proximales Ende 9 aufweist. Das proximale Ende 9 ist hier offen ausgebildet und dient zur Aufnahme zumindest eines Teils eines vereinfacht, jedoch nicht näher bezeichneten Aufnahmebehälters, der auch als Blutentnahmeröhrchen bezeichnet werden kann.

Im Bereich des distalen Endes 8 des Hauptkörperteils 6 ist ein Halteansatz 10 angeordnet bzw. ausgebildet. Der Halteansatz 10 kann zur Aufnahme und Halterung der Nadelanordnung 3 und/oder der Schutzvorrichtung 4 dienen bzw. dazu ausgebildet sein. Bevorzugt wird das distale Ende 8 zumindest bereichsweise von einer hier vereinfacht dargestellt Stirnwand 11 verschlossen.

Dabei sei hier allgemein erwähnt, dass in der vorliegenden Anmeldung mit der Bezeichnung "distal" stets jener Teil der Sicherheitsbaugruppe 1 bezeichnet wird, welcher bei der bestimmungsgemäßen Verwendung durch einen Benutzer (medizinisches Personal) von diesem abgewendet und somit dem Patienten zugewendet ist. Als "proximal" wird stets jener Teil der Sicherheitsbaugruppe 1 bezeichnet, welcher bei der bestimmungsgemäßen Verwendung durch einen Benutzer (medizinisches Personal) diesem zugewendet und somit vom Patienten abgewendet ist. Dabei können einzelne die Sicherheitsbaugruppe 1 bildenden Bauteile in Richtung der Längsachse 7 gesehen jeweils eine dem distalen Ende 8 oder dem proximalen Ende 9 zugewendete Seite aufweisen.

Die Nadelanordnung 3 umfasst bei dem hier gezeigten Ausführungsbeispiel eine Kanüle 12, einen an dieser angeordneten Halteteil 13, welcher in Richtung der Längsachse 7 gesehen zwischen einem distalen Kanülenende 14 und einem davon distanzierten proximalen Kanülenende 15 angeordnet ist. Eine Durchgangsöffnung zwischen den beiden Kanülenenden 14, 15 verbindet diese miteinander und stellt während der bestimmungsgemäßen Verwendung eine Strömungsverbindung her. Dadurch ist ein Durchfluss durch die hohl ausgebildete Kanüle 12 möglich.

Der Halteansatz 10 dient als Kupplungselement einer Kupplungsvorrichtung, mit welchem der Halteteil 13 der Nadelanordnung 3 verbunden bzw. damit gekuppelt werden kann. Im vorliegenden Ausführungsbeispiel erfolgt dies über eine Gewindeanordnung, wie dies hinlänglich bekannt ist. Dabei kann auch ein mehrgängiges Gewinde Anwendung finden. Der Halteansatz 10 ist dabei in etwa rohrförmig ausgebildet und trägt an seiner Innenseite bzw. Innenfläche einen Teil der Gewindeanordnung. Dadurch kann die Nadelanordnung 3 bedarfsweise lösbar an der Handhabungsvorrichtung 2 gehalten werden.

Ist die Kanüle 12 - wie hier beim vorliegenden Ausführungsbeispiel gezeigt - als doppelendige Hohlnadel ausgebildet, überragt ein Teilbereich der Kanüle 12 - nämlich ihr distales Kanülenende 14 - die Handhabungsvorrichtung 2 auf die von der Aufnahmekammer 5 abgewendete Seite. Weiters ist noch im Bereich des distalen Kanülenendes 14 vereinfacht dargestellt, dass in an sich bekannter Weise durch Abschrägung der Kanüle 12 eine Öffnung 16 ausgebildet wird, welche durch die schräge Ausrichtung in Bezug zur Längsachse 7 annähernd die Form einer Ellipse bzw. ein Ovals ausbildet. Dadurch weist die Öffnung 16 eine längere sowie kürzere Öffnungsachse auf.

Die miteinander in Eingriff stehenden Gewinde der Gewindeanordnung sind durch ein am Basisteil 19 angeordnetes Innengewinde und am Halteteil 13 der Nadelanordnung 3 durch ein damit in Eingriff bringbares Außengewinde gebildet. Bevorzugt wird die Gewindeanordnung durch ein doppelgängiges Gewinde gebildet, wodurch eine Einsetzmöglichkeit und eine damit verbundene Endposition der Öffnung 16 an der Kanüle 12 jeweils um 180 ° zueinander erzielt.

Die Schutzvorrichtung 4 weist zumindest ein in einer Schwenkebene 17 verschwenkbar ausgebildetes Schutzelement 18 auf, wobei dieses von einer ersten, den Teilbereich der Kanüle 12 freigebenden Stellung (Freigabestellung) in eine zweite, den Teilbereich abdeckenden Stellung (Schutzstellung) verschwenkbar ausgebildet ist.

Zur Anbringung derselben an der Handhabungsvorrichtung 2 weist die Schutzvorrichtung 4 einen in etwa rohrförmig bzw. ringförmig ausgebildeten Basisteil 19 auf, mit dem diese am Halteansatz 10 der Handhabungsvorrichtung 2 gegebenenfalls lösbar gehalten werden kann. Der Halteansatz 10 bildet mit dem Basisteil 19 eine Halte- bzw. Arretiervorrichtung 20 aus. Weiters ist der Basisteil 19 am Halteansatz 10 der Handhabungsvorrichtung 2 um die Längsachse 7 drehbar daran gehalten.

Durch die Arretiervorrichtung 20 ist eine Montage und damit das Anbringen der Schutzvorrichtung 4, insbesondere des Basisteils 19 am Halteansatz 10 möglich. Es könnte damit aber auch eine bedarfsweise Abnahme bzw. Trennung der schwenkbaren Schutzvorrichtung 4 von der Handhabungsvorrichtung 2 erfolgen. Bevorzugt wird eine vormontierte Sicherheitsbaugruppe 1 bereitgestellt, bei welcher an der Handhabungsvorrichtung 2 sowohl die Schutzvorrichtung 4 als auch die Nadelanordnung 3 in der jeweiligen Gebrauchsstellung gehalten sind. Eine nachträgliche Trennung ist aus Sicherheitsgründen nicht mehr vorgesehen, wobei nach dem bestimmungsgemäßen Gebrauch und bei sich in der Schutzstellung befindlichem Schutzelement 18 die gesamte Sicherheitsbaugruppe 1 dem Entsorgungsvorgang zugeführt wird. Dabei können die zusammenwirkenden Bauteile derart zueinander ausgebildet sein, dass eine gegenseitige Halterung durch eine Rastverbindung realisiert ist, jedoch die Drehbarkeit des Basisteils 19 um die Längsachse 7 und somit um den Halteansatz 10 möglich ist. Eine Verlagerung in axialer Richtung ist nicht oder nur in einem eher geringen Ausmaß möglich.

Ist der Halteansatz 10 am Hauptkörperteil 6 der Handhabungsvorrichtung 2 durch einen in etwa rohrförmigen Bauteil gebildet, kann eine Mittelachse dieses Bauteils deckungsgleich zur Längsachse 7 ausgerichtet verlaufend sein. Zumeist ist der Halteansatz 10 konzentrisch zur Längsachse 7 des Hauptkörperteils 6 im Bereich des zumindest bereichsweise verschlossenen distalen Endes 8 an der Stirnwand 11 feststehend angeordnet. Diese mittige bzw. zentrale Anordnung in Bezug zum Hauptkörperteil 6 findet bei einer doppelendig ausgebildeten Kanüle 12 Anwendung, wobei dann deren distales Kanülenende 14, wie bereits zuvor beschrieben, die Stirnwand 11 auf die von der Aufnahmekammer 5 abgewandte Richtung überragt und das proximale Kanülenenden 15 bei an der Handhabungsvorrichtung 2 eingesetzter Stellung in die Aufnahmekammer 5 in Richtung auf das offene, proximale Ende 9 hin hineinragt. Diese Anordnung der Kanüle 12 in Bezug zur Handhabungsvorrichtung 2 ist in dieser Form allgemein bekannt. Eine nicht näher bezeichnete flexibel ausgebildete Schutzhülle kann den in die Aufnahmekammer 5 hineinragenden Teilabschnitt der Kanüle 12 abdecken und bei Herstellen einer Strömungsverbindung mit dem Innenraum eines Abnahmeröhrchens durchstochen und verlagert werden.

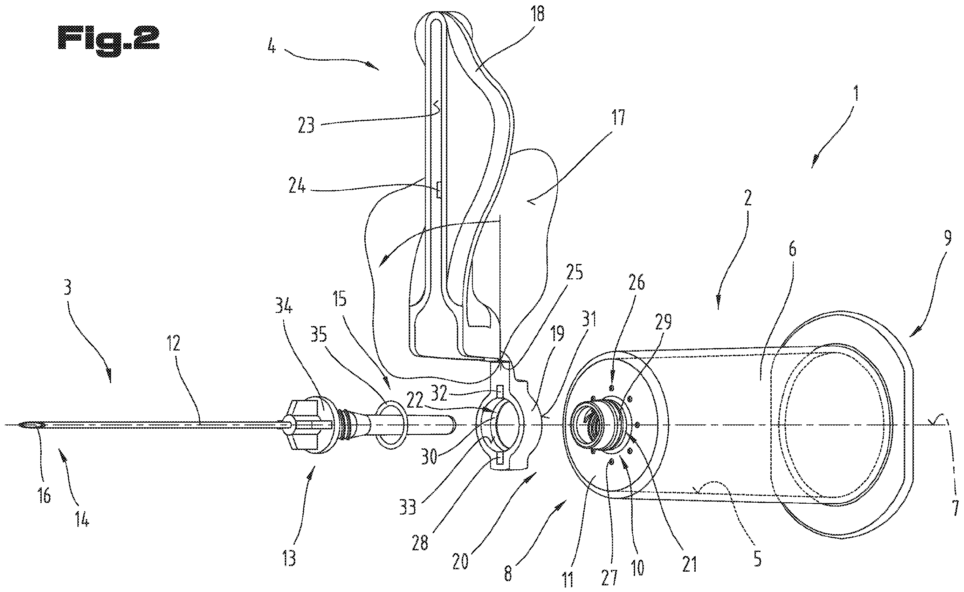

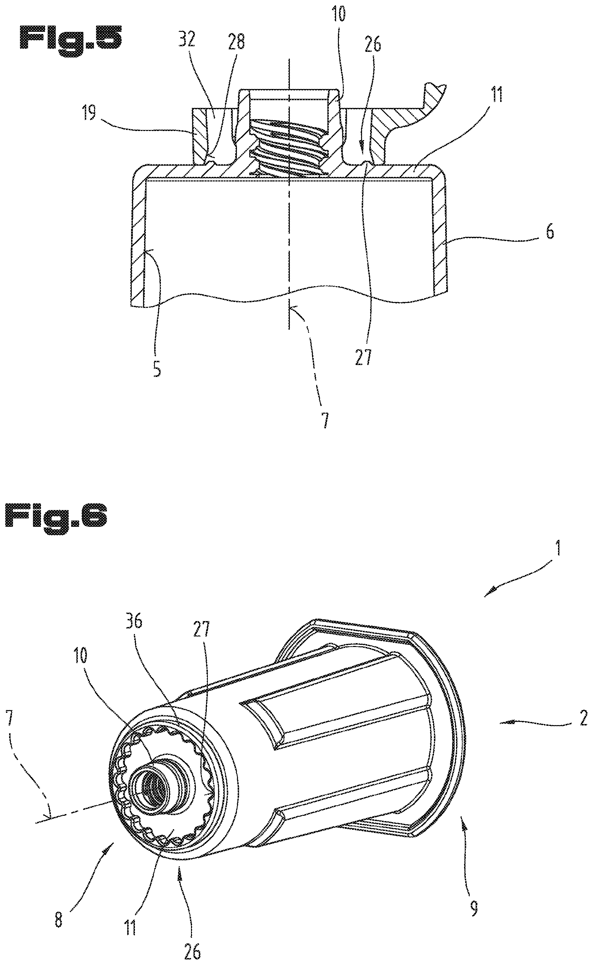

Der Halteansatz 10 überragt die Stirnwand 11 auf die von der Aufnahmekammer 5 abgewendete Seite. Der in etwa rohr- bzw. ringförmig ausgebildete Basisteil 19 übergreift den Halteansatz 10 im Bereich seiner äußeren Oberfläche, insbesondere im Bereich des Zylindermantels, zumindest bereichsweise. So entspricht eine äußere Abmessung bzw. ein äußerer Durchmesser des Halteansatzes 10 annähernd der inneren Abmessung bzw. dem inneren Durchmesser des Basisteils 19, wodurch aufgrund der gewählten Abmessungen der Basisteil 19 über den Halteansatz 10 in axialer Richtung aufschieb- bzw. aufsetzbar ist. Um ein unbeabsichtigtes Lösen der beiden Bauteile in der in Eingriff stehenden Stellung zu verhindern, kann die Arretiervorrichtung 20 weiters noch miteinander in Eingriff bringbare Arretierelemente 21, 22 umfassen, wie diese am besten aus einer Zusammenschau der Fig. 2 und 5 zu ersehen sind. Diese beiden Arretierelemente 21, 22 können zumindest bereichsweise über den Umfang des Halteansatzes 10 bzw. des Basisteils 19 an den jeweils einander zugewandten Seiten angeordnet sein. Es können der Halteansatz 10 sowie der Basisteil 19 aber auch jede andere Raumform wie z.B. oval, mehreckig usw. aufweisen.

Bei diesem hier gezeigten Ausführungsbeispiel ist das erste Arretierelement 21 - siehe Fig. 2 - am Halteansatz 10, nämlich dem hier dargestellten rohrförmigen Bauteil durch einen den Halteansatz 10 auf die von der Längsachse 7 abgewendete Seite radial überragenden Wulst 29 gebildet, der zumindest bereichsweise aber auch durchlaufend über den Umfang des Haltensatzes 10 verlaufend angeordnet sein kann.

Das weitere Arretierelement 22 ist am rohr- bzw. ringförmigen Basisteil 19 auf der der Längsachse 7 zugewendeten Seite die Innenfläche überragenden, durch einen weiteren Wulst 30 gebildet, wie dies am besten aus der Fig. 2 und 5 ersehen ist. Dieser, das weitere Arretierelement 22 bildende Wulst 30 kann wiederum zumindest bereichsweise über den inneren Umfang des Basisteils 19 verlaufend angeordnet sein, wobei in der eingerasteten Stellung das weitere Arretierelement 22 das am Halteansatz 10 angeordnete erste Arretierelement 21 auf der der Stirnwand 11 zugewendeten Seite übergreift.

Durch diese hier nur beispielhaft für eine Vielzahl von weiteren möglichen Ausbildungen der zusammenwirkenden Arretierelemente 21, 22 beschriebene Ausführungsform wird erreicht, dass bei gefügten bzw. in Eingriff stehendem Basisteil 19 und Halteansatz 10 eine unbeabsichtigte Bewegung der beiden Bauteile in Richtung der Längsachse 7 relativ zueinander verhindert ist. Aufgrund dieser zuvor beschriebenen Ausbildung der zusammenwirkenden Arretierelemente 21, 22 ist bei ortsfester Halterung der Handhabungsvorrichtung 2 jedoch eine Schwenkung bzw. Verdrehung der Schutzvorrichtung 4 relativ gegenüber der Handhabungsvorrichtung 2 um die Längsachse 7 möglich. Es kann das Arretierelement 21 aber auch z. B. durch eine oder mehrerer nutförmig ausgebildete Vertiefungen am Halteansatz 10 und das weitere Arretierelemente 22 durch einen oder mehrere damit zusammenwirkende bzw. darin eingreifende Vorsprünge am Basisteil 19 ausgebildet sein.

Wie noch vereinfacht aus der Fig. 2 zu ersehen ist, weist das Schutzelement 18 einen Kanal 23 zur Aufnahme zumindest eines Teilbereiches der Kanüle 12 im Bereich von deren distalen Kanülenenden 14 auf. Um ein unbeabsichtigtes Zurückschwenken des Schutzelements 18 aus der Schutzstellung zu verhindern, ist im Bereich des Kanals 23 mindestens ein Rückhalteelement 24 zur einrastenden Halterung am Teilbereichs der Kanüle 12 in der Schutzstellung angeordnet, wie dies bereits aus dem Stand der Technik bekannt ist.

Zur Erzielung der Schwenkbewegung des Schutzelements 18 relativ gegenüber dem Basisteil 19 kann zwischen diesen Bauteilen ein elastisch verformbarer Steg angeordnet sein. Zur exakteren Festlegung der Schwenkbewegung und der Stellung des Schutzelementes 18 in der den Teilbereich der Kanüle 12 abdeckenden Schutzstellung ist es Vorteilhaft, wenn das Schutzelement 18 in einer senkrecht zur Schwenkebene 17 ausgerichteten Schwenkachse 25 verschwenkbar ist, wobei diese Schwenkachse 25 bevorzugt durch ein Filmscharnier ausgebildet sein kann.

Weiters ist eine Rastvorrichtung 26 mit zumindest einem ersten Rastelement 27 an der Handhabungsvorrichtung 2 sowie zumindest einem zweiten Rastelement 28 am Basisteil 19 der Schutzvorrichtung 4 vorgesehen, wobei bei in Eingriff stehenden ersten und zweiten Rastelementen 27, 28 eine relative Position der Schutzvorrichtung 4, insbesondere deren Schutzelement 18, bezüglich der Handhabungsvorrichtung 2 festgelegt ist. Dies betrifft die Stellung des Schutzelements 18 bezüglich der Winkellage um die Längsachse 7.

Durch die in der Raststellung miteinander zusammenwirkenden Rastelemente 27, 28 kann bei entsprechender gegenseitiger Ausrichtung stets ein Blickkontakt zur in etwa elliptisch bzw. oval ausgebildeten Öffnung 16 eingestellt werden, wie dies bei der Verwendung der Sicherheitsbaugruppe 1 für das Abnehmen von Körperflüssigkeiten und/oder das Abgeben von fluiden Stoffen in den Körper für medizinische Zwecke notwendig ist. Zumeist wird die Handhabungsvorrichtung 2, die Schutzvorrichtung 4 bzw. die daraus gebildete medizinische Sichterheitsbaugruppe 1 auf dem medizinischen Gebiet der Blutabnahme eingesetzt.

Damit wird es für den Anwender der Sicherheitsbaugruppe 1 möglich, das Schutzelement 18 der Schutzvorrichtung 4 an die Anwendungsbedingungen und seine persönlichen Bedürfnisse individuell anpassen und einstellen zu können. Weiters kann so beispielsweise bei einer Blutabnahme das Schutzelement 18 bei der bestimmungsgemäßen Verwendung so verstellt werden, dass dieses nicht dem Arm des Patienten zugewendet ist und somit bei Durchführung einer Abnahme hinderlich ist. So kann stets ein Einblick bzw. die Betrachtung der Öffnung 16 sichergestellt werden, welche für die Durchführung des Einstichvorganges in einen Körperteil, wie beispielsweise einer Vene oder einer Ader, notwendig ist, um einen bestin-nnungsgemäßen Einstichvorgang durchführen zu können.

Vorteilhaft ist bei dieser medizinischen Sicherheitsbaugruppe 1, dass die Schutzvorrichtung 4 unabhängig von der Nadelanordnung 3 bereits an der Handhabungsvorrichtung 2 vormontiertbar ist und erst bei bestimmungsgemäßer Verwendung die Nadelanordnung 3 an der Handhabungsvorrichtung 2 anzubringen ist - im vorliegenden Fall eingeschraubt werden kann. Derüber hinaus kann aber auch die Nadelanordnung 3 bereits vormontiert an der Handhabungsvorrichtung 2 befestigt sein. Dadurch werden weitere Eügevorgänge vermieden und so die Gefahr von Stichverletzungen vermieden. Unabhängig davon wäre es aber auch noch möglich, die Kanüle 12 direkt mit dem Hauptkörperteil 6, insbesondere dessen Halteansatz 10, zu verbinden. Dies kann z.B. durch einen Klebevorgang, Schweißvorgang oder ähnliche Verbindungsvorgänge erfolgen.

Während des bestimmungsgemäßen Gebrauches ist das Schutzelement 18 seitlich der Handhabungsvorrichtung 2 angeordnet, ohne dass dabei eine hinderliche Stellung entweder zwischen dem Arm des Patienten und der Handhabungsvorrichtung 2 oder ein Blickkontakt zu der dem Anwender, beispielsweise einem Arzt, zugewendeten Öffnung 16 der Kanüle 12 behindert wird.

Die hier beschriebene Handhabungsvorrichtung 2, die Schutzvorrichtung 4 sowie der Halteteil 13 an der Kanüle 12 werden zumeist aus einem Kunststoff hergestellt, wobei die Handhabungsvorrichtung 2 bevorzugt transparent bzw. durchsichtig ausgebildet ist, um einen Einblick in den Innenraum zu ermöglichen.

Wie bereits zuvor beschrieben, ist der Basisteil 19 der Schutzvorrichtung 4 drehbar am Halteansatz 10 der Handhabungsvorrichtung 2 gelagert bzw. daran gehalten. Um eine freie Drehbarkeit zu vermeiden und eine voreinstellbare, relative Positionierung des Schutzelements 18 in Bezug zum Anschliff der Kanüle 12, nämlich der Öffnung 16, individuell einstellen zu können, ist die Rastvorrichtung 26 vorgesehen. So ist bei diesem hier gezeigten ersten Ausführungsbeispiel das zumindest erste an der Handhabungsvorrichtung 2 vorgesehene Rastelement 27 an der Stirnwand 11 angeordnet. Das zumindest zweite am Basisteil 19 der Schutzvorrichtung 4 vorgesehene Rastelement 28 ist im Bereich einer der der Stirnwand 11 zugewendeten Stirnfläche 31 des Basisteils 19 angeordnet bzw. daran ausgebildet. Dabei kann es sich um eine Ausnehmung, Vertiefung oder dgl. handeln. Im vorliegenden Ausführungsbeispiel ist das zumindest erste Rastelement 27 an der Stirnwand 11 in axialer Richtung vorragend von dieser ausgebildet bzw. angeordnet. So kann das erste Rastelement 27 ausgewählt sein aus der Gruppe von Noppen, Stirnzähnen, Vorsprüngen, Ausnehmungen. Es kann aber auch das zweite Rastelement 28 die Stirnfläche 31 in axialer Richtung überragen und in eine dazu in der Stirnwand 11 angeordnete Vertiefung eingreifen.

Um über den Umfang gesehen mehrere Raststellungen individuell festlegen zu können, können auch mehrere erste Rastelemente 27 umfänglich verteil an der Stirnwand 11 der Handhabungsvorrichtung 2 angeordnet bzw. vorgesehen sein.

Zur Ausbildung des zweiten Rastelements 28 am Basisteil 19 der Schutzvorrichtung 4 ist hier vorgesehen, dass der Basisteil 19 an seiner der Stirnwand 11 zugewendeten Stirnfläche 31 zumindest eine Ausnehmung 32 aufweist. Damit bildet diese in der Stirnfläche 31 vorgesehene Ausnehmung 32 das zumindest eine zweite Rastelement 28 aus. Das erste Rastelement 27 stützt sich bei der entsprechenden verrasteten Position an zumindest einer der die Ausnehmung 32 in Umfangsrichtung begrenzenden Seitenwand ab.

Im vorliegenden Ausführungsbeispiel ist gezeigt, dass der Basisteil 19 der Schutzvorrichtung 4 an seiner der Längsachse 7 zugewendeten Innenfläche 33 zumindest zwei diametral gegenüberliegend angeordnete Ausnehmungen 32 aufweist. Diese Ausnehmungen 32 erstrecken sich im vorliegenden Ausführungsbeispiel durchgehend in axialer Richtung zwischen dem distalen Endbereich und dem proximalen Endbereich des Basisteils 19. So können die Ausnehmungen 32 schlitz- bzw. nutförmig ausgebildet sein.

Durch die Anordnung bzw. das Vorsehen der schlitzförmige Ausnehmungen 32 kann eine gewisse zusätzliche Elastizität des Basisteils 19 für die Aufsetzbewegung auf den Halteansatz 10 der Handhabungsvorrichtung 2 erzielt werden. Damit kann eine größere Nachgiebigkeit während des Aufschieben- bzw. Aufsetzvorganges erreicht werden, wobei nach dem in Eingriff bringen der zuvor beschriebenen Arretierelemente 21, 22 ein ausreichend fester, jedoch drehbarer Sitz des Basisteils 19 am Halteansatz 10 erreicht wird. Damit übernehmen die Ausnehmungen 32 eine Doppelfunktion und können mit den an der Stirnwand 11 angeordneten, ersten Rastelementen 27 bei entsprechender, gegenseitiger Ausrichtung bzw. relativen Winkellage miteinander in Rastverbindung gebracht werden.

Weiters ist hier noch dargestellt, dass die Nadelanordnung 3, insbesondere deren Halteteil 13 einen insbesondere flanschförmig ausgebildeten Ansatz 34 aufweist, welcher in der gekuppelten bzw. eingeschraubten Stellung der Nadelanordnung 3 in den Halteansatz 10 der Handhabungsvorrichtung 2 den Basisteil 19 in radialer Richtung zumindest teilweise übergreift. Der Ansatz 34 kann auch als Haltetlansch bezeichnet werden. Damit wird eine Abnahme der Schutzvorrichtung 4 von der Handhabungsvorrichtung 2 verhindert. Dies ist vor allem dann von Bedeutung, wenn das Schutzelement 18 in die abdeckende Schutzstellung über das nach der Verwendung abzudeckende, distale Kanülenende 14 verschwenkt und mit dem Rückhalteelement 24 gegen eine neuerliche Verwendung gesichert ist. Ansonsten wäre es möglich, dass zwar das Schutzelement 18 in einer verrasteten bzw. verriegelten Stellung an der Kanüle 12 in der Schutzstellung gehalten ist, jedoch die gesamte Schutzvorrichtung 4 von der Handhabungsvorrichtung 2 in axialer Richtung abgezogen werden kann. Dies wird durch das Übergreifen des Basisteils 19 vom Ansatz 34 verhindert.

Durch eine entsprechende maßliche Abstimmung des Halteansatzes 10, des Basisteils 19 sowie des axialen Abstandes zwischen der Stirnwand 11 und der dieser zugewendeten Anlagefläche des Ansatzes 34 kann im Zusammenwirken mit den in Eingriff stehenden Arretierelementen 21, 22 das axiale Spiel des Basisteils 19 sowie der in Eingriff stehenden Rastelemente 27, 28 beeinflusst werden.

Weiters ist hier noch im Bereich der Nadelanordnung 3 dargestellt, dass zwischen dem Ansatz 34 und dem Basisteil 19 ein schematisch angedeutetes Federelement 35 angeordnet sein kann, welches vereinfacht durch einen kreisförmigen bzw. torusförmigen Bauteil dargestellt ist. Als Federelement 35 wird hier jedes Bauteil verstanden, welches zumindest in Axialrichtung kompressibel ausgebildet ist und bei axialer Verformung bzw. Vorspannung eine Druckkraft aufbaut bzw. abgibt. So könnte das Federelement 35 als flexibler Ring aus den unterschiedlichsten Werkstoffen oder aber auch als Druckfeder, Fächerscheibe, Zahnscheibe oder dgl. ausgebildet sein. Durch dieses Federelement 35 kann ein gewisses, axiales Spiel des Basisteils 19 am Halteansatz 10 ermöglicht werden. Das Federelement 35 erzeugt dann eine entsprechende Anpresskraft in axialer Richtung auf den Basisteil 19, welcher mit seiner Stirnfläche 31 an die Stirnwand 11 angedrückt wird. Da die Rastvorrichtung 26 mit ihren Rastelementen 27, 28 in einer Ebene gegenüberliegend zueinander angeordnet sind, kann so auch noch die Feststellkraft der Rastvorrichtung 26 beeinflusst werden. Die Rastkraft ist dabei in axialer Richtung zwischen den Rastelementen 27, 28 wirkend.

In der Fig. 6 bis 8 ist eine weitere und gegebenenfalls für sich eigenständige Ausführungsform der Sicherheitsbaugruppe 1 gezeigt, wobei wiederum für gleiche Teile gleiche Bezugszeichen bzw. Bauteilbezeichnungen wie in den vorangegangenen Fig. 1 bis 5 verwendet werden. Um unnötige Wiederholungen zu vermeiden, wird auf die detaillierte Beschreibung in den vorangegangenen Fig. 1 bis 5 hingewiesen bzw. Bezug genommen.

Diese weitere hier gezeigte Möglichkeit zur Ausbildung der Rastvorrichtung 26 mit ihren ersten und zweiten Rastelementen 27, 28 kann ebenfalls wiederum dazu benutzt werden, um die relative Drehlage des Schutzelements 18 der Schutzvorrichtung 4 in Umfangsrichtung bezüglich der Längsachse 7 festlegen zu können. Auch diese hier gezeigte Sicherheitsbaugruppe 1 umfasst wiederum die Handhabungsvorrichtung 2 mit dem hohl ausgebildeten Hauptkörperteil 6, welcher das distale Ende 8 sowie das proximale Ende 9 aufweist. Im Bereich des distalen Endes 8 kann wiederum die Stirnwand 11 ausgebildet sein, von welcher in axialer Richtung vorragend der Halteansatz 10 daran angeordnet ist. Das proximale Ende 9 ist offen ausgebildet und dient zur Aufnahme zumindest eines Teils eines Aufnahmebehälters, welcher beispielsweise ein evakuiertes Probenentnahmeröhrchen ist, um Körperflüssigkeit darin aufnehmen zu können.

Die Schutzvorrichtung 4 weist auch hier wiederum den rohrförmigen bzw. ringförmigen Basisteil 19 auf, mit welchem das Schutzelement 18 schwenkbar verbunden ist.

Im Gegensatz zu der zuvor beschriebenen Ausbildung der Rastvorrichtung 26 ist hier vorgesehen, dass das zumindest erste an der Handhabungsvorrichtung 2 vorgesehene Rastelement 27 in radialer Richtung bezüglich des Halteansatzes 10 distanziert von diesem auf der von der Längsachse 7 abgewendeten Seite angeordnet ist. Um über den Umfang gesehen mehrere unterschiedlich zueinander ausgerichtete Raststellungen zu erzielen, können auch hier wiederum mehrere, erste Rastelemente 27 umfänglich verteilt an der Handhabungsvorrichtung 2 angeordnet bzw. ausgebildet sein. Dabei kann es sich um einzelne voneinander getrennt angeordnete Rastelemente 27 handeln, welche zueinander in Umfangsrichtung distanziert bzw. beabstandet voneinander angeordnet sind.

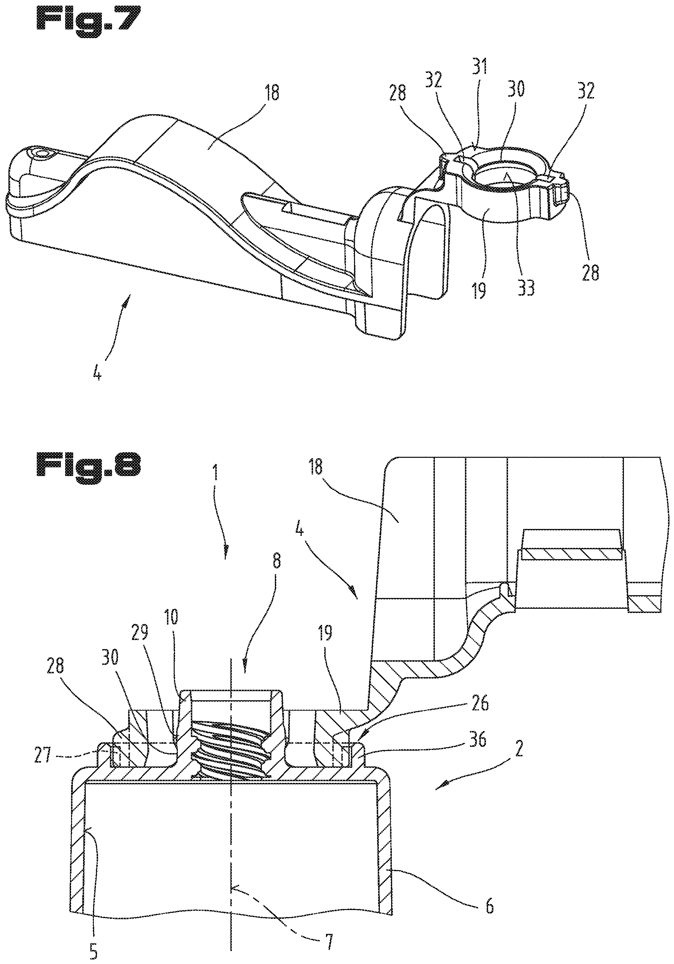

Das oder die ersten Rastelemente 27 sind durch deren radiale Distanzierung vom Halteansatz 10 weiters auch noch vom Basisteil 19 der Schutzvorrichtung 4 in radialer Richtung davon distanziert an der Handhabungsvorrichtung 2 angeordnet bzw. dort ausgebildet, wie dies aus einer Zusammenschau der Fig. 6 und 8 zu ersehen ist. Somit ist das oder sind die ersten Rastelemente 27 der Außenseite des Basisteils 19 gegenüberliegend angeordnet und damit dem äußeren Umfang des Basisteils 19 zugewendet. So ist in radialer Richtung gesehen der Basisteil 19 zwischen dem Halteansatz 10 und dem oder den ersten Rastelemente 27 angeordnet. Der Basisteil 19 ist trotz der außenseitig angeordneten Rastvorrichtung 26 an seiner der Längsachse 7 zugewendeten Innenseite am Halteansatz frei drehbar gelagert.

Im vorliegenden Ausführungsbeispiel sind mehrere der ersten Rastelemente 27 an einem umfänglich durchlaufend ausgebildeten, rohrförmigen bzw. kranzförmigen Ansatz 36 angeordnet oder an diesem ausgebildet. So bilden die ersten Rastelemente 27 eine Art wellenförmige Innenverzahnung aus. In Umfangsrichtung gesehen wird durch diese Wellenform ein unterschiedlicher, radialer Abstand bezüglich der Längsachse 7 ausgebildet, wobei das erste Rastelement 27 entweder durch die Vertiefung mit dem größeren Radialabstand oder durch die hin auf die Längsachse 7 gerichtete Erhöhung zwischen den Vertiefungen gebildet ist.

So weist der hier dargestellte Hauptkörperteil 6 der Handhabungsvorrichtung 2 an seinem distalen Ende 8 nicht nur die senkrecht bezüglich der Längsachse 7 ausgerichtete Stirnwand 11 mit dem daran angeordneten und von dieser in axialer Richtung vorragenden Halteansatz 10 sondern auch das mindestens erste Rastelement 27 auf. Die Ausrichtung und Wirkungsrichtung der Rastkraft zwischen den Rastelementen 27, 28 erfolgt dabei in einer senkrecht zur Längsachse 7 ausgerichteten Ebene in radialer Richtung bezüglich der Längsachse 7.

Wie nun besser aus der Fig. 7 zu ersehen ist, ist das zumindest zweite am Basisteil 19 vorgesehene Rastelement 28 vom Basisteil 19 in radialer Richtung vorragend ausgebildet. Bei aufgesetzter bzw. gekuppelter Schutzvorrichtung 4 mit der Handhabungsvorrichtung 2 ragt im vorliegenden Ausführungsbeispiel das weitere Rastelement 28 jeweils in das als Vertiefung vorgesehene erste Rastelement 27 in der verrasteten Position hinein. Die dreh- bzw. schwenkbare Lagerung des Basisteils 19 erfolgt wiederum am Halteansatz 10 der Handhabungsvorrichtung 2.

Auch dieser hier gezeigte Basisteil 19 der Schutzvorrichtung 4 weist an seiner der Längsachse 7 zugewendeten Innenfläche 33 wiederum die zumindest zwei diametral gegenüber liegenden schlitzförmig ausgebildeten Ausnehmungen 32 auf. Diese unterbrechen den ringförmigen Basisteils 19 im Bereich seiner Innenfläche 33 als umfänglich durchlaufenden Bauteil. Diese in einer Axialebene symmetrisch bzw. spiegelbildlich zueinander ausgebildeten Basisteilhälften werden im Bereich der Ausnehmungen 32 jeweils auf der von der Längsachse 7 abgehendeten Seite durch einen stegartigen Bauteil miteinander verbunden. An diesem stegartigen Bauteil ist dann das bzw. sind die weiteren Rastelemente 28 angeordnet bzw. daran ausgebildet. Das oder die zweiten Rastelemente 28 sind somit auf einer von der Längsachse 7 abgewendeten Außenfläche 37 des Basisteils 19 angeordnet. Bei Vorsehen der Ausnehmungen 32 sind die zweiten Rastelemente 28 jeweils gegenüberliegend bezüglich der Ausnehmungen 32 angeordnet.

Die Halterung bzw. Arretierung des Basisteils 19 am Halteansatz 10 kann auch hier wiederum durch die zuvor beschriebene Arretiervorrichtung 20 im Zusammenwirken der ersten und zweiten Arretierelemente 21, 22 erfolgen, wie dies bereits zuvor detailliert beschrieben worden ist.

Dadurch, dass hier die während des Verstellvorganges um die Längsachse 7 zu überwindende Rastkraft in radialer Richtung wirkt, kann der Basisteil 19 mit relativ geringem, axialem Spiel am Halteansatz 10 drehbar gelagert bzw. gehalten sein. Die in axialer Richtung wirkende Haltekraft kann dabei durch die Arretiervorrichtung 20 mit ihren Arretierelementen 21, 22 und/oder über die Halterung des in radialer Richtung vorragenden Ansatzes 34 am Halteteil 13 der Nadelanordnung 3 am Halteansatz 10 und somit der Handhabungsvorrichtung 2 erfolgen.

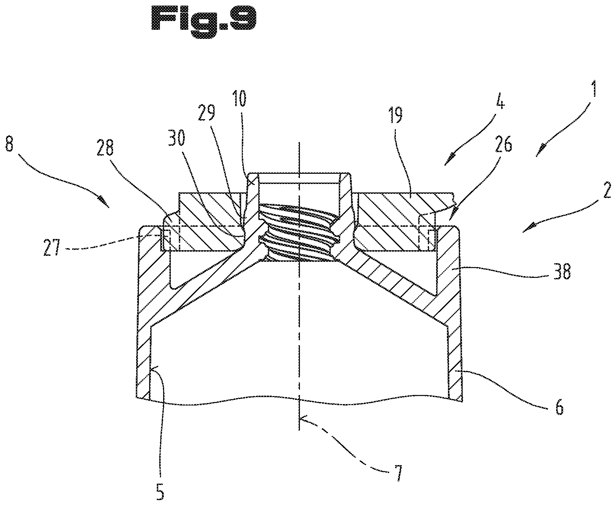

In der Fig. 9 ist eine weitere und gegebenenfalls für sich eigenständige Ausführungsform der Handhabungsvorrichtung 2 zur Bildung der Sicherheitsbaugruppe 1 gezeigt, wobei wiederum für gleiche Teile gleiche Bezugszeichen bzw. Bauteilbezeichnungen wie in den vorangegangenen Fig. 1 bis 8 verwendet werden. Um unnötige Wiederholungen zu vermeiden, wird auf die detaillierte Beschreibung in den vorangegangenen Fig. 1 bis 8 hingewiesen bzw. Bezug genommen.

Die hier gezeigte Ausführungsform der Rastvorrichtung 26 ist in ihrer Wirkungsweise und Anordnung ähnlich aufgebaut, wie diese zuvor in den Fig. 6 bis 8 beschrieben worden ist. Die Schutzvorrichtung 4, insbesondere der Basisteil 19, kann wiederum gleich ausgebildet sein, dieser in den Fig. 6 bis 8 beschrieben worden ist.

Der Unterschied besteht hier lediglich in der Anordnung und Ausbildung der weiteren Rastelemente 28 an der Handhabungsvorrichtung 2. Der Halteansatz 10 ist hier nicht an einer senkrecht zur Längsachse 7 ausgerichteten Stirnwand 11 angeordnet, sondern ist über einen gegenüber dem Hauptkörperteil 6 sich hin zum distalen Ende 8 verjüngenden Übergangsabschnitt damit verbunden. Zur Anordnung bzw. Ausbildung der weiteren Rastelemente 28 ist hier am Hauptkörper 6 im Bereich seines distalen Endes 8 ein in etwa konzentrisch bezüglich des Halteansatzes 10 angeordneter und in axialer Richtung vom Hauptkörperteil 6 vorragender Kragen 38 vorgesehen. Der Kragen 38 ist in radialer Richtung bezüglich des Halteansatzes 10 distanziert von diesem auf der von der Längsachse 7 abgewendeten Seite angeordnet. An diesem ist das mindestens zweite Rastelement 28 angeordnet oder daran ausgebildet. Die Ausbildung der zweiten bzw. weiteren Rastelemente 28 kann dabei wieder analog erfolgen, wie dies bereits zuvor in der Fig. 5 gezeigt und beschrieben worden ist.

Der Ordnung halber sei anschließend darauf hingewiesen, dass zum besseren Verständnis des Aufbaus der Sicherheitsbaugruppe 1 diese bzw. deren Bestandteile teilweise unmaßstäblich und/oder vergrößert und/oder verkleinert dargestellt wurden.

Die den eigenständigen erfinderischen Lösungen zugrundeliegende Aufgabe kann der Beschreibung entnommen werden.

Sämtliche Angaben zu Wertebereichen in gegenständlicher Beschreibung sind so zu verstehen, dass diese beliebige und alle Teilbereiche daraus mit umfassen, z.B. ist die Angabe 1 bis 10 so zu verstehen, dass sämtliche Teilbereiche, ausgehend von der unteren Grenze 1 und der oberen Grenze 10 mitumfasst sind, d.h. sämtliche Teilbereich beginnen mit einer unteren Grenze von 1 oder größer und enden bei einer oberen Grenze von 10 oder weniger, z.B. 1 bis 1,7, oder 3,2 bis 8,1 oder 5,5 bis 10.

Die Ausführungsbeispiele zeigen mögliche Ausführungsvarianten der der Sicherheitsbaugruppe 1 , wobei an dieser Stelle bemerkt sei, dass die Erfindung nicht auf die speziell dargestellten Ausführungsvarianten derselben eingeschränkt ist, sondern vielmehr auch diverse Kombinationen der einzelnen Ausführungsvarianten untereinander möglich sind und diese Variationsmöglichkeit aufgrund der Lehre zum technischen Handeln durch gegenständliche Erfindung im Können des auf diesem technischen Gebiet tätigen Fachmannes liegt. Es sind also auch sämtliche denkbaren Ausführungsvarianten, die durch Kombinationen einzelner Details der dargestellten und beschriebenen Ausführungsvariante möglich sind, vom Schmutzumfang mit umfasst. Weiters können auch Einzelmerkmale oder Merkmalskombinationen aus den gezeigten und beschriebenen unterschiedlichen Ausführungsbeispielen für sich eigenständige, erfinderische oder erfindungsgemäße Lösungen darstellen.

Vor allem können die einzelnen in den Fig. 1 bis 5; 6 bis 8; 9 gezeigten Ausführungen den Gegenstand von eigenständigen, erfindungsgemäßen Lösungen bilden. Die diesbezüglichen, erfindungsgemäßen Aufgaben und Lösungen sind den Detailbeschreibungen dieser Figuren zu entnehmen.

### Bezugszeichenaufstellung

- 1: Sicherheitsbaugruppe
- 2: H an dhabung svorri ch tun g
- 3: Nadelanordnung
- 4: Schutzvorrichtung
- 5: Aufnahmekammer

- 6: Hauptkörperteil
- 7: Längsachse
- 8: distales Ende
- 9: proximales Ende 10 Halteansatz

- 11: Stirnwand
- 12: Kanüle
- 13: Halteteil
- 14: distales Kanülenende
- 15: proximales Kanülenende

- 16: Öffnung
- 17: Schwenkebene
- 18: Schutzelement
- 19: Basisteil
- 20: Arretiervorrichtung

- 21: Arretierelement
- 22: Arretierelement
- 23: Kanal
- 24: Rückhalteelement
- 25: Schwenkachse

- 26: Rastvorrichtung
- 27: Rastelement
- 28: Rastelement
- 29: Wulst
- 30: Wulst

- 31: Stirnfläche
- 32: Ausnehmung
- 33: Innenfläche
- 34: Ansatz
- 35: Federelement

- 36: Ansatz
- 37: Außenfläche
- 38: Kragen

## Patentansprüche

1. Sicherheitsbaugruppe (1) für die Medizintechnik, insbesondere die Blutabnahme, umfassend eine Handhabungsvorrichtung (2) mit einem hohl ausgebildeten Hauptkörperteil (6), welcher ein distales Ende (8) mit einem Halteansatz (10) und ein offen ausgebildetes proximales Ende (9) aufweist, das zur Aufnahme zumindest eines Teils eines Aufnahmebe hälters ausgebildet ist, und sich zwischen dem distalen Ende (8) und dem proximalen Ende (9) eine Längsachse (7) erstreckt, eine Schutzvorrichtung (4) mit einem ringförmigen Basisteil (19) sowie einem mit dem Basisteil (19) schwenkbar verbundenen Schutzelement (18), welches von einer Freigabestellung in eine Schutzstellung verschwenkbar ist, um in der Schutzstellung eine an der Handhabungsvorrichtung (2) anbringbare Nadelanordnung (4) zumindest teilweise abzudecken, wobei der Basisteil (19) am Halteansatz (10) der Handhabungsvorrichtung (2) um die Längsachse (7) drehbar gehalten ist, eine Rastvorrichtung (26) mit zumindest einem ersten Rastelement (27) an der Handhabungsvorrichtung (2) sowie zumindest einem zweiten Rastelement (28) an der Schutzvorrichtung (4), wobei bei in Eingriff stehenden ersten und zweiten Rastelementen (27, 28) eine relative Position der Schutzvorrichtung (4) bezüglich der Handhabungsvorrichtung (2) festgelegt ist, und wobei das zumindest erste an der Handhabungsvorrichtung (2) vorgesehene Rastelement (27) bezüglich des Halteansatzes (10) in radialer Richtung bezüglich der Längsachse (7) distanziert vom Halteansatz (10) sowie in radialer Richtung bezüglich der Längsachse (7) auf der von der Längsachse (7) abgewendeten Seite des Basisteils (19) angeordnet ist, **dadurch gekennzeichnet, dass** das zumindest zweite Rastelement (28) am Basisteil (19) der Schutzvorrichtung (4) an der von der Längsachse (7) abgewendeten Seite des Basisteils (19) an diesem angeordnet oder ausgebildet ist und das zumindest zweite Rastelement (28) vom Basisteil (19) in radialer Richtung vorragend ausgebildet ist, wobei eine zwischen den Rastelementen (27, 28) aufgebaute Rastkraft bei sich in der Raststellung befindlichen ersten und zweiten Rastelementen (27, 28) durch Verdrehung mit einer Verstellkraft überwunden ist und dabei der Basisteil (19) am Halteansatz (10) der Handhabungsvorrichtung (2) um die Längsachse (7) frei verdrehbar ist.

2. Sicherheitsbaugruppe (1) nach Anspruch 1, **dadurch gekennzeichnet, dass** mehrere erste Rastelemente (27) umfänglich verteilt an der Handhabungsvorrichtung (2) angeordnet sind.

3. Sicherheitsbaugruppe (1) nach Anspruch 2, **dadurch gekennzeichnet, dass** die ersten Rastelemente (27) an einem umfänglich durchlaufend ausgebildeten rohrförmigen Ansatz (36) angeordnet oder daran ausgebildet sind.

4. Sicherheitsbaugruppe (1) nach einem der vorhergehenden Ansprüche, **dadurch gekennzeichnet, dass** am Hauptkörperteil (6) im Bereich seines distalen Endes (8) ein konzentrisch bezüglich des Halteansatzes (10) angeordneter und in axialer Richtung vorragender Kragen (38) vorgesehen ist, der in radialer Richtung bezüglich des Halteansatzes (10) distanziert von diesem auf der von der Längsachse (7) abgewendeten Seite angeordnet ist und das mindestens erste Rastelement (27) am Kragen (38) angeordnet oder daran ausgebildet ist.

5. Sicherheitsbaugruppe (1) nach einem der Ansprüche 1 bis 3, **dadurch gekennzeichnet, dass** der Hauptkörperteil (6) der Handhabungsvorrichtung (2) an seinem distalen Ende (8) eine in senkrechter Richtung bezüglich der Längsachse (7) ausgerichtete Stirnwand (11) aufweist, an welcher sowohl der Halteansatz (10) als auch das mindestens erste Rastelement (27) angeordnet oder daran ausgebildet sind.

6. Sicherheitsbaugruppe (1) nach einem der vorhergehenden Ansprüche, **dadurch gekennzeichnet, dass** der Basisteil (19) der Schutzvorrichtung (4) an seiner der Längsachse (7) zugewendeten Innenfläche (33) zumindest zwei diametral gegenüberliegende, schlitzförmig ausgebildete Ausnehmungen (32) aufweist, wobei auf einer von der Längsachse (7) abgewendeten Außenfläche (37) des Basisteils (19) jeweils gegenüberliegend bezüglich der Ausnehmung (32) das zweite Rastelement (28) angeordnet ist.

7. Sicherheitsbaugruppe (1) für die Medizintechnik, insbesondere die Blutabnahme, umfassend eine Handhabungsvorrichtung (2) mit einem hohl ausgebildeten Hauptkörperteil (6), welcher ein distales Ende (8) mit einem Halteansatz (10) und ein offen ausgebildetes proximales Ende (9) aufweist, das zur Aufnahme zumindest eines Teils eines Aufnahmebehälters ausgebildet ist, und sich zwischen dem distalen Ende (8) und dem proximalen Ende (9) eine Längsachse (7) erstreckt, eine Schutzvorrichtung (4) mit einem ringförmigen Basisteil (19) sowie einem mit dem Basisteil (19) schwenkbar verbundenen Schutzelement (18), welches von einer Freigabestellung in eine Schutzstellung verschwenkbar ist, um in der Schutzstellung eine an der Handhabungsvorrichtung (2) anbringbare Nadelanordnung (4) zumindest teilweise abzudecken, wobei der Basisteil (19) am Halteansatz (10) der Handhabungsvorrichtung (2) um die Längsachse (7) drehbar sowie in Axialrichtung gehalten ist, eine Rastvorrichtung (26) mit zumindest einem ersten Rastelement (27) an der Handhabungsvorrichtung (2) sowie zumindest einem zweiten Rastelement (28) am Basisteil (19) der Schutzvorrichtung (4), wobei bei in Eingriff stehenden ersten und zweiten Rastelementen (27, 28) eine relative Position der Schutzvorrichtung (4) bezüglich der Handhabungsvorrichtung (2) festgelegt ist, wobei der Hauptkörperteil (6) der Handhabungsvorrichtung (2) an seinem distalen Ende (8) eine in senkrechter Richtung bezüglich der Längsachse (7) ausgerichtete Stirnwand (11) aufweist, an welcher der Halteansatz (10) in axialer Richtung vorragend von dieser angeordnet ist, **dadurch gekennzeichnet, dass** das zumindest erste an der Handhabungsvorrichtung (2) vorgesehene Rastelement (27) bezüglich des Halteansatzes (10) in radialer Richtung bezüglich der Längsachse (7) distanziert vom Halteansatz (10) an der Stirnwand (11) angeordnet und in axialer Richtung von dieser vorragend ausgebildet ist und das zumindest zweite am Basisteil (19) der Schutzvorrichtung (4) vorgesehene Rastelement (28) im Bereich einer der der Stirnwand (11) zugewendeten Stirnfläche (31) des Basisteils (19) angeordnet ist, und dass die ersten und zweiten Rastelemente (27, 28) der Rastvorrichtung (26) in einer Ebene gegenüberliegend zueinander angeordnet sind, wobei eine zwischen den Rastelementen (27, 28) aufgebaute Rastkraft bei sich in der Raststellung befindlichen ersten und zweiten Rastelementen (27, 28) in axialer Richtung wirkt und diese Rastkraft durch Verdrehung mit einer Verstellkraft überwunden ist und dabei der Basisteil (19) am Halteansatz (10) der Handhabungsvorrichtung (2) um die Längsachse (7) frei verdrehbar ist.

8. Sicherheitsbaugruppe (1) nach Anspruch 7, **dadurch gekennzeichnet, dass** das mindestens erste Rastelement (27) ausgewählt ist aus der Gruppe von Noppe, Stirnzahn, Vorsprung, Ausnehmung.

9. Sicherheitsbaugruppe (1) nach Anspruch 7 oder 8, **dadurch gekennzeichnet, dass** mehrere erste Rastelemente (27) umfänglich verteilt an der Stirnwand (11) der Handhabungsvorrichtung (2) angeordnet sind.

10. Sicherheitsbaugruppe (1) nach einem der Ansprüche 7 bis 9, **dadurch gekennzeichnet, dass** der Basisteil (19) der Schutzvorrichtung (4) an seiner der Stirnwand (11) zugewendeten Stirnfläche (31) zumindest eine Ausnehmung (32) aufweist, welche das zweite Rastelement (28) bildet.

11. Sicherheitsbaugruppe (1) nach einem der Ansprüche 7 bis 9, **dadurch gekennzeichnet, dass** der Basisteil (19) der Schutzvorrichtung (4) an seiner der Längsachse (7) zugewendeten Innenfläche (33) zumindest zwei diametral gegenüberliegende, schlitzförmig ausgebildete Ausnehmungen (32) aufweist, wobei die Ausnehmungen (32) die zweiten Rastelemente (28) bilden.

12. Sicherheitsbaugruppe (1) nach einem der Ansprüche 1 bis 6 oder 7 bis 11, **dadurch gekennzeichnet, dass** diese weiters noch eine Nadelanordnung (3) mit einer Kanüle (12) mit in Richtung der Längsachse (7) voneinander distanziert angeordneten distalen und proximalen Kanülenenden (14,15) sowie einen zwischen den Kanülenenden (14,15) an der Kanüle (12) angeordneten Halteteil (13) umfasst, der in der Gebrauchsstellung mit dem Halteansatz (10) der Handhabungsvorrichtung (2) verbunden ist und am Halteteil (13) ein insbesondere flanschförmig ausgebildeter Ansatz (34) angeordnet ist, welcher den Basisteil (19) in radialer Richtung zumindest teilweise übergreift.

13. Sicherheitsbaugruppe (1) nach Anspruch 12, **dadurch gekennzeichnet, dass** zwischen dem Ansatz (34) am Halteteil (13) der Nadelanordnung (3) und dem Basisteil (19) der Schutzvorrichtung (4) ein Federelement (35) angeordnet ist.

## Claims

1. Safety assembly (1) for medical technology, in particular for taking blood samples, comprising a handling device (2) having a main part (6) of hollow design which has a distal end (8) with a holding attachment (10) and a proximal end (9) of open design which is designed to accommodate at least a part of a container, and a longitudinal axis (7) extends between the distal end (8) and the proximal end (9), a protective device (4) having an annular base part (19) and a protective element (18) pivotally connected to the base part (19) which can be pivoted from a released position into a guard position so that in the guard position it at least partially covers a needle assembly (4) which can be attached to the handling device (2), wherein the base part (19) is retained on the holding attachment (10) of the handling device (2) so as to be rotatable about the longitudinal axis (7), a locking device (26) having at least one first detent element (27) on the handling device (2) and at least one second detent element (28) on the protective device (4), wherein a relative position of the protective device (4) is defined with respect to the handling device (2) when the first and second detent elements (27, 28) are engaged, and wherein the at least first detent element (27) provided on the handling device (2) is arranged at a distance from the holding attachment (10) in the radial direction with respect to the longitudinal axis (7) and in the radial direction with respect to the longitudinal axis (7) on the side of the base part (19) facing away from the longitudinal axis (7), **characterised in that** the at least second detent element (28) on the base part (19) of the protective device (4) is arranged or formed on the side of the base part (19) facing away from the longitudinal axis (7) and the at least second detent element (28) is disposed projecting out from the base part (19) in the radial direction, wherein a locking force generated between the detent elements (27, 28) when the first and second detent elements (27, 28) are in the locked position is overcome by rotation with a displacement force, and the base part (19) is thus freely rotatable about the longitudinal axis (7) on the holding attachment (10) of the handling device (2).

2. Safety assembly (1) according to claim 1, **characterised in that** several first detent elements (27) are distributed around the circumference of the handling device (2).

3. Safety assembly (1) according to claim 2, **characterised in that** the first detent elements (27) are disposed or formed on a circumferentially continuously extending neck (36) of tubular design.

4. Safety assembly (1) according to one of the preceding claims, **characterised in that** a collar (38) arranged concentrically with respect to the holding attachment (10) and projecting axially is provided in the region of the distal end (8) of the main part (6) and is arranged at a distance in the radial direction from the holding attachment (10) on the side facing away from the longitudinal axis (7) and the at least first detent element (27) is disposed or formed on the collar (38).

5. Safety assembly (1) according to one of claims 1 to 3, **characterised in that** the main part (6) of the handling device (2) has an end wall (11) at its distal end (8) which is aligned perpendicularly with respect to the longitudinal axis (7) and on which both the holding attachment (10) and the at least first detent element (27) are disposed or formed.

6. Safety assembly (1) according to one of the preceding claims, **characterised in that** the base part (19) of the protective device (4) has at least two diametrically opposite, slot-shaped recesses (32) on its internal face (33) facing the longitudinal axis (7), wherein the second detent element (28) is disposed on an external face (37) of the base part (19) facing away from the longitudinal axis (7) opposite each corresponding recess (32).

7. Safety assembly (1) for medical technology, in particular for taking blood samples, comprising a handling device (2) having a main part (6) of hollow design which has a distal end (8) with a holding attachment (10) and a proximal end (9) of open design which is designed to accommodate at least a part of a container, and a longitudinal axis (7) extends between the distal end (8) and the proximal end (9), a protective device (4) having an annular base part (19) and a protective element (18) pivotally connected to the base part (19) which can be pivoted from a released position into a guard position so that in the guard position it at least partially covers a needle assembly (4) which can be attached to the handling device (2), wherein the base part (19) is retained on the holding attachment (10) of the handling device (2) in the axial direction and so as to be rotatable about the longitudinal axis (7), a locking device (26) having at least one first detent element (27) on the handling device (2) and at least one second detent element (28) on the base part (19) of the protective device (4), wherein a relative position of the protective device (4) is defined with respect to the handling device (2) when the first and second detent elements (27, 28) are engaged, wherein the distal end (8) of the main part (6) of the handling device (2) has an end wall (11) aligned perpendicularly to the longitudinal axis (7), on which wall the holding attachment (10) is arranged protruding axially therefrom, **characterised in that** the at least first detent element (27) provided on the handling device (2) is arranged on the end wall (11) with respect to the holding attachment (10) in the radial direction, with respect to the longitudinal axis (7) at a distance from the holding attachment (10) and designed to protrude axially therefrom, and the at least second detent element (28) provided on the base part (19) of the protective device (4) is arranged in the area of an end face (31) of the base part (19) facing towards the end wall (11), and that the first and second detent elements (27, 28) of the locking device (26) are arranged opposite one another in one plane, wherein a locking force generated between the detent elements (27, 28) when the first and second detent elements (27, 28) are in the locked position is exerted in the axial direction and this locking force is overcome by rotation with a displacement force, and the base part (19) is thus freely rotatable about the longitudinal axis (7) on the holding attachment (10) of the handling device (2).

8. Safety assembly (1) according to claim 7, **characterised in that** the at least first detent element (27) is selected from the group comprising a stud, radial tooth, projection, recess.

9. Safety assembly (1) according to claim 7 or 8, **characterised in that** multiple first detent elements (27) are distributed around the circumference of the end wall (11) of the handling device (2).

10. Safety assembly (1) according to one of claims 7 to 9, **characterised in that** the base part (19) of the protective device (4) has at least one recess (32) constituting the second detent element (28) on the end face (31) thereof facing the end wall (11).

11. Safety assembly (1) according to one of claims 7 to 9, **characterised in that** the base part (19) of the protective device (4) has at least two diametrically opposite, slot-shaped recesses (32) on its internal face (33) facing the longitudinal axis (7), wherein the recesses (32) constitute the second detent elements (28).

12. Safety assembly (1) according to one of claims 1 to 6 or 7 to 11, **characterised in that** it further comprises a needle assembly (3) having a cannula (12) with distal and proximal cannula ends (14, 15) spaced apart from one another in the direction of the longitudinal axis (7) and a retaining part (13) disposed between the cannula ends (14, 15) on the cannula (12), which retaining part (13) is connected to the holding attachment (10) of the handling device (2) when in the operating position, and a neck (34) constructed particularly in the form of a flange is disposed on the retaining part (13) and at least partially extends over the base part (19) in the radial direction.

13. Safety assembly (1) according to claim 12, **characterised in that** a spring element (35) is disposed between the neck (34) on the retaining part (13) of the needle assembly (3) and the base part (19) of the protective device (4).

## Revendications

1. Groupe de sécurité (1) pour la technique médicale, en particulier pour prélever du sang, comprenant un dispositif de manipulation (2) ayant une partie de corps principale (6) d'une conception creuse qui a une extrémité distale (8) ayant une attache de support (10) et une extrémité proximale (9) d'une conception ouverte qui est conçue pour accueillir au moins une partie d'un conteneur de réception, un axe longitudinal (7) s'étendant entre l'extrémité distale (8) et l'extrémité proximale (9), un dispositif de protection (4) ayant une partie de base de forme annulaire (19) et un élément de protection (18) connecté de manière pivotante à la partie de base (19), pouvant pivoter depuis une position libérée jusque dans une position de protection pour couvrir au moins partiellement un ensemble à aiguille (4) qui peut être placé sur le dispositif de manipulation (2) dans une position de protection, la partie de base (19) étant retenue sur l'attache de support (10) du dispositif de manipulation (2) de manière à pouvoir tourner autour de l'axe longitudinal (7), un dispositif d'encliquetage (26) ayant au moins un premier élément d'encliquetage (27) sur le dispositif de manipulation (2) et au moins un second élément d'encliquetage (28) sur le dispositif de protection (4), une position relative du dispositif de protection (4) étant définie par rapport au dispositif de manipulation (2) lorsque les premier et second éléments d'encliquetage (27, 28) sont mis en prise, et le au moins un premier élément d'encliquetage (27) prévu sur le dispositif de manipulation (2) est agencé espacé de l'attache de support (10) dans une direction radiale par rapport à l'axe longitudinal (7) et dans une direction radiale par rapport à l'axe longitudinal (7) sur le côté de la partie de base (19) dirigé à l'opposé de l'axe longitudinal (7), dans lequel le au moins un second élément d'encliquetage (28) sur la partie de base (19) du dispositif de protection (4) est agencé ou formé sur la partie de base (19) sur son côté dirigé à l'opposé de l'axe longitudinal (7), et le au moins un second élément d'encliquetage (28) est réalisé faisant saillie à partir de la partie de base (19) dans une direction radiale, et une force d'encliquetage générée entre les éléments d'encliquetage (27, 28) lorsque les premier et second éléments d'encliquetage (27, 28) sont dans la position encliquetée est surmontée par une rotation à l'aide d'une force motrice, et la partie de base (19) est ainsi montée sur l'attache de support (10) du dispositif de manipulation (2) de manière à pouvoir tourner librement autour de l'axe longitudinal (7).

2. Groupe de sécurité (1) selon la revendication 1, **caractérisé en ce que** plusieurs premiers éléments d'encliquetage (27) sont répartis circonférentiellement autour du dispositif de manipulation (2).

3. Groupe de sécurité (1) selon la revendication 2 **caractérisé en ce que** les premiers éléments d'encliquetage (27) sont disposés ou formés sur une attache s'étendant circonférentiellement (36) d'une conception tubulaire.

4. Groupe de sécurité (1) selon l'une quelconque des revendications précédentes, **caractérisées en ce qu'**un col (38) faisant saillie dans une direction axiale est fourni sur la partie de corps principale (6) agencé d'une manière concentrique par rapport à l' attache de support (10) dans la zone de son extrémité distale (8), qui est espacé dans une direction radiale par rapport à l'attache de support (10) loin de celle-ci sur le côté dirigé à l'opposé de l'axe longitudinal (7), et le au moins un premier élément d'encliquetage (27) est disposé ou formé sur le col (38).

5. Groupe de sécurité (1) selon l'une quelconque des revendications 1 à 3, **caractérisé en ce que** la partie de corps principale (6) du dispositif de manipulation (2) a une paroi d'extrémité (11) au niveau de son extrémité distale (8) orientée dans une direction perpendiculaire par rapport à l'axe longitudinal (7), sur laquelle l'attache de support (10) et le au moins premier élément d'encliquetage (27) sont disposés ou formés.

6. Groupe de sécurité (1) selon l'une quelconque des revendications précédentes, **caractérisées en ce que** la partie de base (19) du dispositif de protection (4) a au moins deux évidements en forme de fente diamétralement opposés (32) sur sa surface interne (33) faisant face à l'axe longitudinal (7), le second élément d'encliquetage (28) étant disposé sur une surface externe (37) de la partie de base (19) dirigée à l'opposé de l'axe longitudinal (7) s'étendant respectivement opposé à l'évidement (32).

7. Groupe de sécurité (1) pour la technique médicale, en particulier pour prélever du sang, comprenant un dispositif de manipulation (2) ayant une partie de corps principale (6) d'une conception creuse qui a une extrémité distale (8) ayant une attache de support (10) et une extrémité proximale (9) d'une conception ouverte qui est conçue pour accueillir au moins une partie d'un conteneur de réception, un axe longitudinal (7) s'étendant entre l'extrémité distale (8) et l'extrémité proximale (9), un dispositif de protection (4) ayant une partie de base de forme annulaire (19) et un élément de protection (18) connecté de manière pivotante à la partie de base (19), pouvant pivoter depuis une position libérée jusque dans une position de protection pour couvrir au moins partiellement un ensemble à aiguille (4) qui peut être placé sur le dispositif de manipulation (2) dans une position de protection, la partie de base (19) étant retenue sur l'attache de support (10) du dispositif de manipulation (2) de manière à pouvoir tourner autour de l'axe longitudinal (7), ainsi que dans une direction axiale, un dispositif d'encliquetage (26) ayant au moins un premier élément d'encliquetage (27) sur le dispositif de manipulation (2) et au moins un second élément d'encliquetage (28) sur la partie de base (19) du dispositif de protection (4), une position relative du dispositif de protection (4) étant définie par rapport au dispositif de manipulation (2) lorsque les premier et second éléments d'encliquetage (27, 28) sont mis en prise, la partie de corps principale (6) du dispositif de manipulation (2) a une paroi d'extrémité (11) au niveau de son extrémité distale (8) orientée dans une direction perpendiculaire par rapport à l'axe longitudinal (7) sur laquelle l'attache de support (10) est disposée faisant saillie à partir de celle-ci dans une direction axiale, **caractérisé en ce que** le au moins un premier élément d'encliquetage (27) prévu sur le dispositif de manipulation (2) est agencé par rapport à l'attache de support (10) espacé de l'attache de support (10) dans la direction radiale par rapport à l'axe longitudinal (7) sur la paroi d'extrémité (11), et fait saillie à partir de là dans une direction axiale, et le au moins second élément d'encliquetage (28) prévu sur la partie de base (19) du dispositif de protection (4) est agencé dans la zone d'une surface d'extrémité (31) de la partie de base (19) faisant face à la paroi d'extrémité (11), et **en ce que** les premier et second éléments d'encliquetage (27, 28) du dispositif d'encliquetage (26) sont disposés s'étendant opposés l'un à l'autre dans un plan, et lorsque les premier et second éléments d'encliquetage (27, 28) sont dans la position encliquetée, une force d'encliquetage générée entre les éléments d'encliquetage (27, 28) agit dans une direction axiale, et cette force d'encliquetage est surmontée par une rotation à l'aide d'une force motrice, et la partie de base (19) est ainsi montée sur l'attache de support (10) du dispositif de manipulation (2) de manière à pouvoir tourner librement autour de l'axe longitudinal (7).

8. Groupe de sécurité (1) selon la revendication 7, **caractérisé en ce que** le au moins un premier élément d'encliquetage (27) est sélectionné dans le groupe comprenant un goujon, une denture frontale, une saillie, un évidement.

9. Groupe de sécurité (1) selon la revendication 7 ou 8, **caractérisé en ce que** plusieurs premiers éléments d'encliquetage (27) sont répartis circonférentiellement autour de la paroi d'extrémité (11) du dispositif de manipulation (2).

10. Groupe de sécurité (1) selon l'une quelconque des revendications 7 à 9, **caractérisé en ce que** la partie de base (19) du dispositif de protection (4) a au moins un évidement (32) constituant le second élément d'encliquetage (28) sur sa surface d'extrémité (31) tournée vers la paroi d'extrémité (11).

11. Groupe de sécurité (1) selon l'une quelconque des revendications 7 à 9, **caractérisé en ce que** la partie de base (19) du dispositif de protection (4) présente au moins deux évidements en forme de fente diamétralement opposés (32) sur sa surface interne (33) tournée vers l'axe longitudinal (7), les évidements (32) constituant les seconds éléments d'encliquetage (28).

12. Groupe de sécurité (1) selon l'une quelconque des revendications 1 à 6 ou 7 à 11, **caractérisé en ce qu'**il comporte en outre un ensemble à aiguille (3) ayant une canule (12) avec des extrémités de canule distale et proximale (14, 15) agencées espacées à une distance l'une de l'autre dans la direction de l'axe longitudinal (7), et une partie de support (13) agencée sur la canule (12) entre les extrémités de canule (14, 15), qui est connectée à l'attache de support (10) du dispositif de manipulation (2) dans la position d'utilisation, et une attache (34) d'une conception en particulier en forme de bride est agencée sur la partie de support (13), qui s'étend au moins partiellement vers l'extérieur depuis la partie de base (19) dans une direction radiale.

13. Groupe de sécurité (1) selon la revendication 12, **caractérisé en ce qu'**un élément ressort (35) est agencé entre l'attache (34) sur la partie de support (13) de l'ensemble à aiguille (3) et la partie de base (19) du dispositif de protection (4).
